(19) Europäisches Patentamt
European Patent Office
Office européen des brevets

(11) Numéro de publication: **0 296 935**
**B1**

(12) **FASCICULE DE BREVET EUROPEEN**

(45) Date de publication du fascicule du brevet: **16.01.91**

(21) Numéro de dépôt: **88401454.9**

(22) Date de dépôt: **13.06.88**

(51) Int. Cl.$^5$: **C 07 C 229/12,**
C 10 M 133/08 // C10N30/06

(54) **Composés polyfluorés, leur préparation et leur utilisation comme additifs pour lubrifiants.**

(30) Priorité: **19.06.87 FR 8708663**

(43) Date de publication de la demande:
**28.12.88 Bulletin 88/52**

(45) Mention de la délivrance du brevet:
**16.01.91 Bulletin 91/03**

(84) Etats contractants désignés:
**AT BE CH DE ES FR GB GR IT LI LU NL SE**

(56) Documents cités:
**EP-A-0 083 077**
**EP-A-0 248 697**
**FR-A-2 520 377**
**US-A-4 409 001**

(73) Titulaire: **ATOCHEM**
**4 & 8, Cours Michelet La Défense 10**
**F-92800 Puteaux (FR)**

(72) Inventeur: **Germanaud, Laurent**
**79 Route Neuve**
**F-69540 Irigny (FR)**
Inventeur: **Hermant, Marc**
**10 Ter, Rue Lamartine**
**F-95240 Cormeilles-en-Parisis (FR)**

(74) Mandataire: **Leboulenger, Jean et al**
**ATOCHEM Département Propriété Industrielle**
**F-92091 Paris la Défense 10 Cédex 42 (FR)**

**EP 0 296 935 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne le domaine des produits fluorés et celui des lubrifiants. Elle a plus particulièrement pour objet de nouveaux composés fluorés utilisables come additifs anti-usure pour lubrifiants.

Il est déjà connu d'incorporer certains dérivés organofluorés dans les lubrifiants en vue d'améliorer leurs propriétés anti-usure. Ainsi, par exemple, dans le brevet FR 2 520 377 on a proposé l'incorporation d'amines ou d'amino-alcools à chaîne polyfluorée; cependant, bien que ces composés permettent d'obtenir des compositions lubrifiantes possédant des propriétés anti-usure et un pouvoir réducteur du frottement remarquables, leur utilisation est souvent limité en raison de leur volatilité élevée qui entraîne une diminution de leur efficacité au cours du temps.

Il a maintenant été trouvé qu'on peut sensiblement remédier à cet inconvénient en utilisant, come additifs fluorés antiusure, les composés répondant à la formule générale:

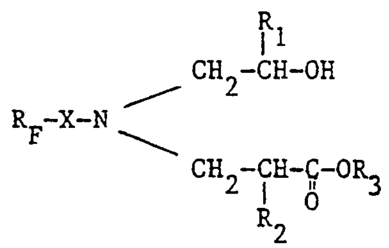

(I)

dans laquelle:

$R_F$ désigne un radical perfluoré, de préférence un radical perfluoroalkyle linéaire ou ramifié contenant de 2 à 20 atomes de carbone,

X représente un enchaînement $—(CH_2CF_2)_a(CH_2)_b—$, $—CF{=}CHCH_2—$ ou $—CFHCH_2CH_2—$,

a est un nombre entier allant de 0 à 10, de préférence de 0 à 3,

b est un nombre entier pouvant aller de 1 à 4, mais est égal à 2 quand a est différent de 0,

$R_1$ représente un atome d'hydrogène ou un radical alkyle, linéaire ou ramifié, contenant de 1 à 12 atomes de carbone,

$R_2$ représente un atome d'hydrogène ou un radical méthyle, et

$R_3$ représente un radical alkyle, linéaire ou ramifié, contenant de 1 à 24 atomes de carbone.

Parmi les composés de formule (I), on préfère plus particulièrement ceux dans lesquels $R_F$ est un radical perfluoroalkyle linéaire contenant de 6 à 16 atomes de carbone, X est un enchaînement $—CH_2CH_2—$, $—CF{=}CHCH_2$ ou $—CFHCH_2CH_2—$, $R_1$ et $R_2$ sont des atomes d'hydrogène, et $R_3$ est un radical alkyle avec 8 à 18 atomes de carbone.

Conformément à la présente invention, on peut utiliser un seul composé de formule (I) ou un mélange de ces composés. Du point de vue économique, il est notamment particulièrement avantageux d'utiliser un mélange industriel de composés ayant des groupements $R_F$, X et/ou $R_3$ différents.

D'une façon générale, les composés de formule (I) peuvent être obtenus par condensation d'un amino-alcool de formule:

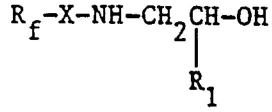

(II)

avec un ester acrylique de formule :

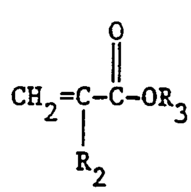

(III)

formules dans lesquelles les symboles $R_F$, X, $R_1$, $R_2$ et $R_3$ ont les mêmes significations que précédemment.

L'addition d'amines non fluorées sur des esters ou amides α, β insaturés est connue depuis longtemps (voir par exemple J. Chem. Soc., 1970, p. 343 et 469 et J. Amer. Chem. Soc., 1949, 2533). Ces méthodes connues peuvent être appliquées à la condensation des amino-alcools de formule (II) sur les esters de formule (III). Cette condensation peut notamment être effectuée a une température comprise entre 20 et 120°C, de préférence entre 20 et 80°C. Elle peut être réalisée en l'absence de solvant, mais est de préférence

menée au sein d'un solvant de l'ester et de l'amino-alcool utilisés. Ce solvant est de préférence un alcool de faible masse moléculaire ($C_1$—$C_4$), mais peut être également choisi parmi les éthers, les nitriles et leurs mélanges (en particulier mélange éther-acétonitrile). La condensation s'effectue bien en l'absence de catalyseur, mais on peut accélérer la réaction en ajoutant un catalyseur acide tel que l'acide acétique ou l'acide sulfurique.

L'amino-alcool fluoré (II) et l'ester (III) sont généralement utilisés en quantités sensiblement équimolaires. Cependant, dans le cas des esters (III) à faible point d'ébullition (par exemple l'acrylate de méthyle), on utilisera avantageusement, un excès d'ester come solvant de la réaction. Cet excès qui peut aller jusqu'à 5 moles par mole d'amino-alcool est, aprés réaction, éliminé par distillation à pression atmosphérique ou sous vide.

Les composés de formule (I) dans lesquels $R_3$ est un radical alkyle à longue chaîne ($C_5$—$C_{24}$) peuvent également être préparés à partir d'un composé de formule (I) où $R_3$ est un radical alkyle inférieur ($C_1$—$C_4$) par transestérification de ce dernier à l'aide d'un alcool à longue chaîne.

Selon un mode réalisation particulier de l'invention, la condensation de l'amino-alcool (II) sur l'ester (III) peut être menée avec un défaut d'ester (III), ce défaut pouvant aller jusqu'à la moitié de la quantité théorique. On obtient alors un mélange comprenant, en plus du composé (I), jusqu'à 50% de l'amino-alcool de départ. De tels mélanges sont aussi utilisables comme additifs anti-usure pour lubrifiants et font donc partie de la présente invention. Il en est de même des mélanges plus complexes obtenus à partir d'un mélange industriel d'amino-alcools (II) et/ou d'un mélange industriel d'esters (III).

Comme exemples d'esters de formule (III), on peut citer plus particulièrement les acrylates ou méthacrylates de méthyle, n-butyle, éthyl-2 hexyle, n-dodécyle, n-tétradécyle, n-hexadécyle et n-octadécyle, les acrylates étant préférés.

A l'exception de ceux dans lesquels X désigne un enchaînement —$CFHCH_2CH_2$—, les amino-alcools (II) sont des produits connus (voir par exemple le brevet FR 1 532 284 et ses additions 93 170, 95 059 et 2 102 753, ainsi que le brevet US 3 535 381) qu'on peut obtenir par condensation d'un dérivé iodé de formule:

$$R_F-(CH_2CF_2)_a(CH_2)_b-I \qquad (IV)$$

avec un amino-alcool de formule:

$$H_2N-CH_2\underset{\substack{|\\R_1}}{CH}-OH \qquad (V)$$

$R_F$, a, b et $R_1$ ayant les mêmes significations que ci-dessus. Il convient de signaler que, lorsqu'on met en oeuvre un iodure du type $R_F$—$CH_2CH_2I$, la condensation conduit généralement à un mélange d'amino-alcools fluorés de formules:

$$R_F-CH_2CH_2-NH-CH_2\underset{\substack{|\\R_1}}{CH}-OH \qquad (II\text{-}a)$$

et

$$R'_F-CF{=}CH-CH_2NH-CH_2\underset{\substack{|\\R_1}}{CH}-OH \qquad (II\text{-}b)$$

où $R'_F$ est un radical perfluoré contenant un atome de carbone de moins que le radical $R_F$. Il est cependant possible, si on le désire, de séparer ces deux amino-alcools par chromatographie gazeuse. On peut également préparer sélectivement l'amino-alcool saturé (II-a) en utilisant un gros excès d'amino-alcool (V). De même, on peut accéder sélectivement à l'amino-alcool insaturé (II-b) en condensant l'aminoalcool (V) sur une oléfine fluorée $R_F$—$CH{=}CH_2$ conformément au brevet US 3 535 381 précité.

Les amino-alcools fluorés (II) dans lesquels X désigne un enchaînement —$CFHCH_2CH_2$— peuvent être obtenus par hydrogénation des amino-alcools fluorés (II-b).

Cette hydrogénation peut être réalisée, par exemple, au sein d'un alcool (de préférence le méthanol ou l'éthanol) en présence d'un catalyseur tel que le nickel de Raney ou le palladium sur charbon, à une température pouvant aller de 25 à 250°C (de préférence, entre 50 et 150°C) et sous une pression d'hydrogène qui peut atteindre 200 bars, mais est de préférence comprise entre 5 et 100 bars.

Cette réaction d'hydrogènation peut également être appliquée à un mélange d'amino-alcools fluorés (II-a) et (II-b) pour obtenir un mélange d'amino-alcools fluorés saturés.

La quantité de composé(s) de formule (I); à incorporer dans une huile lubrifiante pour obtenir une efficacité anti-usure optimale est d'au moins 0,01% par rapport au poids de l'huile et est de préférence comprise entre 0,05 et 0,5%.

L'huile lubrifiante peut être une huile minérale, un hydrocarbure synthétique, une huile synthétique appartenant aux différentes familles suivantes: glycols, éthers de glycols, esters de glycols, les polyoxyalkylène glycols, leurs éthers et leurs esters, les esters d'acides monocarboxyliques ou polycarboxyliques et de monoalcools ou polyalcools, cette énumération n'étant pas limitative.

Lorsqu'on utilise comme bases lubrifiantes, les coupes pétrolières destinées à la fabrication des huiles moteurs, telles que les bases "Neutral Solvent", on associe avantageusement aux dérivés fluoro-organiques de l'invention des additifs dispersants-détergents traditionnels tels que les alkylaryl sulfonates et les alkylphénates de calcium ou de baryum, on des dispersants "sans cendres" comme les dérivés succiniques. Les additifs dispersants-détergents favorisent la solubilisation des additifs fluorés dans l'huile sns porter atteinte aux propriétés anti-usure de ces derniers et sans perdre leur propre pouvoir.

L'addition de dérivés fluorés selon l'invention aux huiles formulées contenant déjà des additifs tels que les alkyldithiophosphates de zinc apporte une amélioration sensible du pouvoir anti-usure et une augmentation de la capacité de charge de ces huiles sans perturbation des propriétés apportées par les autres additifs: dispersivité, détergence, pouvoir anti-corrosion, par exemple.

Le remplacement dans les formulations d'huiles pour moteurs à combustion interne de tout ou partie du dithiophosphate de zinc utilisé comme additif anti-usure par 0,1 à 0,2% de composés organofluorés 2% de composés organofluorés selon l'invention permet d'atteindre un niveau de protection contre l'usure égal ou supérieur à celui obtenu avec cet additif traditionnel.

Les additifs fluorés selon l'invention peuvent donc être utilisés soit en remplacement des alkyldithiophosphates de zinc dans les huiles lubrifiantes pour moteurs à essence ou moteurs diesel, soit en suradditivation dans ces huiles.

Les exemples et les tests suivants illustrent l'invention sans la limiter. Les pourcentages s'entendent en poids, sauf mention contraire.

Exemple 1

Dans un ballon de 100 ml surmonté d'un réfrigérant et sous agitation constante, on ajoute 4 g d'acrylate de méthyle à une solution de 20 g d'un mélange des amino-alcools fluorés $C_8F_{17}$—$CH_2CH_2$—NH—$CH_2CH_2OH$ et $C_7F_{15}CF$=$CHCH_2$—NH—$CH_2CH_2OH$ (respectivement 65 et 35% en moles) dans 10 g d'éthanol. On porte ensuite le mélange à reflux pendant 10 heures, puis on élimine l'éthanol et l'excès d'acrylate de méthyle par évaporation sous vide.

Le résidu (22 g), caractérisé par spectroscopie infrarouge (bande ester: 1730 $cm^{-1}$), correspond aux esters de formules:

$$C_8F_{17}C_2H_4-\underset{\displaystyle C_2H_4OH}{\underset{|}{N}}-C_2H_4COOCH_3 \quad \text{et} \quad C_7F_{15}CF{=}CHCH_2-\underset{\displaystyle C_2H_4OH}{\underset{|}{N}}-C_2H_4COOCH_3$$

et se présente sous forme d'un liquide orangé légèrement trouble à température ambiante, mais bien homogène à 60°C.

Exemple 2

On porte à 100°C pendant 10 heures une solution contenant 20 g du même mélange d'amino-alcools fluorés qu'à l'exemple 1, 5,55 g d'acrylate de n-butyle et 10 g de n-butanol. On filtre ensuite le mélange réactionnel, puis on évapore sous vide le solvant et l'excès d'acrylate de butyle.

Le produit obtenu (23 g) correspond aux esters de formules:

$$C_8F_{17}C_2H_4-\underset{\displaystyle C_2H_4OH}{\underset{|}{N}}-C_2H_4COOC_4H_9 \quad \text{et} \quad C_7F_{15}CF{=}CHCH_2-\underset{\displaystyle C_2H_4OH}{\underset{|}{N}}-C_2H_4COOC_4H_9$$

et se présente sous forme d'un liquide orangé, trouble à température ambiante.

Exemple 3

On répète l'exemple 2, mais en ajoutant 0,1 g d'acide acétique et en chauffant le mélange réactionnel pendant seulement 6 heures.

On obtient le même produit avec un rendement de 91%.

Exemple 4

Dans le même appareillage qu'à l'exemple 1 et sous agitation constante, on ajoute 11,4 g d'acrylate de lauryle à une solution de 23,72 g du même mélange d'amino-alcools fluorés, qu'à l'exemple 1 dans 10 g de n-butanol. On porte ensuite le mélange à 100°C pendant 10 heures, puis on élimine le n-butanol par évaporation sous vide, reprend le résidu par du dichlorométhane, filtre la solution et évapore le dichlorométhane.

Le mélange d'esters de formules:

$$C_8F_{17}C_2H_4-\underset{\displaystyle C_2H_4OH}{\underset{|}{N}}-C_2H_4COOC_{12}H_{25} \quad \text{et} \quad C_7F_{15}CF{=}CHCH_2-\underset{\displaystyle C_2H_4OH}{\underset{|}{N}}-C_2H_4COOC_{12}H_{25}$$

ainsi obtenu se présente sous forme d'un liquide orangé limpide (32,7 g; rendement: 93%).

Exemple 5

On chauffe à 80°C sous agitation pendant 8 heures 20 g d'acrylate de méthyle et 20 g d'un mélange industriel d'aminoalcools fluorés de formules:

$$C_nF_{2n+1}CH_2CH_2-NH-CH_2CH_2OH \quad \text{et} \quad C_{n-1}F_{2n-1}CF{=}CHCH_2-NH-CH_2CH_2OH$$

où n est égal à 6, 8, 10, 12 et 14 dans des pourcentages pondéraux respectifs de 56,2%, 27.2%, 12.3%, 3.7% et 0,6%. Ce mélange dont la masse moléculaire moyenne est de 466 contient environ 65% molaire d'amino-alcools saturés et 35% molaire d'amino-alcools insaturés.

Après évaporation de l'excès d'acrylate de méthyle, on obtient avec un rendement de 91,3% un mélange des esters de formule:

$$C_nF_{2n+1}C_2H_4-\underset{\displaystyle C_2H_4OH}{\underset{|}{N}}-C_2H_4COOCH_3 \quad \text{et} \quad C_{n-1}F_{2n-1}CF{=}CHCH_2-\underset{\displaystyle C_2H_4OH}{\underset{|}{N}}-C_2H_4COOCH_3$$

sous forme d'un liquide limpide jaune, caractérisé par spectroscopie IR (bande ester: 1730 $cm^{-1}$).

Exemple 6

On opère comme à l'exemple 2, mais en remplaçant les amino-alcools en $C_8F_{17}$ et $C_7F_{15}$ par 20 g du mélange industriel d'amino-alcools fluorés défini à l'exemple 5 et en utilisant 6 g d'acrylate butyle.

On obtient ainsi 24,05 g d'un liquide limpide correspondant aux esters de formules:

$$C_nF_{2n+1}C_2H_4-\underset{\displaystyle C_2H_4OH}{\underset{|}{N}}-C_2H_4COOC_4H_9 \quad \text{et} \quad C_{n-1}F_{2n-1}CF{=}CHCH_2-\underset{\displaystyle C_2H_4OH}{\underset{|}{N}}-C_2H_4COOC_4H_9$$

Exemple 7

Dans un erlen de 100 ml équipé d'un réfrigérant et sous agitation constante, on ajoute 7,9 g d'acrylate d'éthyl-2 hexyle à une solution de 20 g du mélange industriel d'amino-alcools fluorés défini à l'exemple 5 dans 10 g de n-butanol, puis on chauffe à 80°C pendant 8 heures.

Aprés élimination du n-butanol par évaporation sous vide, on obtient avec un rendement de 95% un liquide limpide jaune constitué par les esters de formules:

$$C_nF_{2n+1}C_2H_4-\underset{\displaystyle C_2H_4OH}{\underset{|}{N}}-C_2H_4COOCH_2\underset{\displaystyle C_2H_5}{\underset{|}{C}H}-C_4H_9$$

et

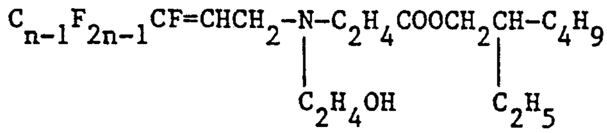

Exemple 8

On opère comme à l'exemple 4, mais en remplaçant les amino-alcools en $C_8F_{17}$ et $C_7F_{15}$ par 20 g du mélange industriel d'amino-alcools fluorés défini à l'exemple 5.

On obtient avec un rendement de 92% un mélange constitué par les esters de formules:

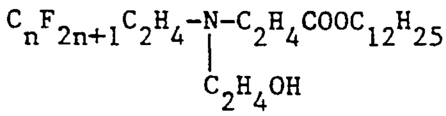 et 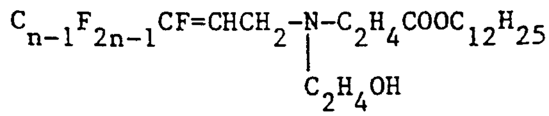

Exemple 9

Dans un flacon contenant 6,18 g du mélange industriel d'amino-alcools fluorés défini à l'exemple 5, on ajoute un défaut d'acrylate de n-butyle (1,09 g, soit 0,65 équivalent molaire) et 9 g de méthanol. On laisse ensuite le mélange sous agitation et à température ambiante pendant 96 heures.

Aprés évaporation du méthanol, on obtient un produit trouble à température ambiante, mais qui devient homogène vers 60°C. Ce produit est constitué, dans une proportion d'environ 60% en moles, par un mélange d'esters de formules:

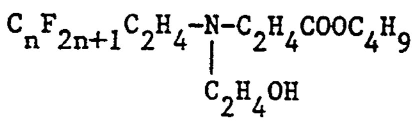 et 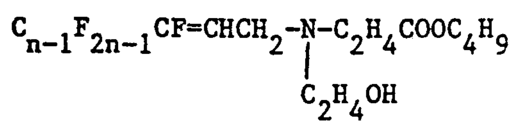

et, dans une proportion d'environ 40% en moles, par un mélange d'aminoalcools fluorés n'ayant pas réagi.

Exemple 10

On répète l'exemple 9, mais en utilisant 1,42 g d'acrylate de n-butyle (0,847 équivalent molaire). On obtient avec un rendement de 90% un produit similaire.

Exemple 11

a/ Dans un autoclave de 4 litres en acier inoxydable, muni d'un système d'agitation à entraînement magnétique, on charge 2000 g d'un mélange des amino-alcools fluorés $C_8F_{17}$—$CH_2CH_2$—NH—$CH_2CH_2OH$ et $C_7F_{15}$—CF=CH—$CH_2$—NH—$CH_2CH_2OH$ (respectivement 67% et 33% en moles), puis 1,2 litre d'éthanol à 99% et 32 g d'une suspension de nickel de Raney à environ 60% dans l'éthanol 99%.

L'autoclave est ensuite purgé trois fois à l'azote sous 30 bars, puis trois fois à l'hydrogène sous 30 bars. On hydrogène ensuite pendant 6 heures et 45 minutes à 70°C, en agitant 2000 tr/min et en maintenant la pression à 20 bars. Après refroidissement, détente et purge de l'autoclave, on filtre le catalyseur, puis on évapore l'éthanol.

On obtient ainsi 1940 g d'un produit solide jaune clair qui fond à 51°C et dont l'analyse CPV donne les résultats suivants:

$C_8F_{17}-CH_2CH_2-NH-CH_2CH_2OH$ .................. 65,6 %

$C_7F_{15}-CFH-CH_2CH_2-NH-CH_2CH_2OH$ ............. 25,4 %

$C_7F_{15}-CH_2CH_2CH_2-NH-CH_2CH_2OH$ .............. 8,9 %

b/ Dans un erlen de 100 ml surmonté d'un réfrigérant et sous agitation constante, on ajoute 1,87 g d'acrylate de n-butyle à une solution de 6,27 g du mélange d'amino-alcools fluorés saturés obtenu ci-dessus dans 10 g de n-butanol. On porte ensuite le mélange à 100°C pendant 10 heures, puis on élimine le n-butanol et l'excès d'acrylate de butyle par évaporation, dissout le résidu jaune dans du dichlorométhane, filtre la solution et évapore le dichlorométhane.

Le mélange d'esters de formule:

$$C_8F_{17}C_2H_4-N(C_2H_4OH)-C_2H_4COOC_4H_9\ ,\quad C_7F_{15}CFHCH_2{=}CH_2-N(C_2H_4OH)-C_2H_4COOC_4H_9$$

et

$$C_7F_{15}C_3H_6-N(C_2H_4OH)-C_2H_4COOC_4H_9$$

ainsi obtenu avec un rendement de 96% se présente sous forme d'un liquide jaune, caractérisé par spectroscopie IR (bande ester à 1725 $cm^{-1}$).

Exemple 12

a/ En opérant comme à l'exemple 11-a, on hydrogène pendant 9 heures à 80°C 2000 g d'un mélange industriel d'amino-alcools fluorés de formules:

$C_nF_{2n+1}-CH_2CH_2-NH-CH_2CH_2OH$ (67 % en moles)

et $C_{n-1}F_{2n-1}-CF{=}CH-CH_2-NH-CH_2CH_2OH$ (33 % en moles)

dont la répartition pondérale des chaînes fluorées est la suivante:

| n | % |
|---|---|
| 6 | 55,7 |
| 8 | 27,2 |
| 10 | 10,15 |
| 12 | 3.9 |
| ≥14 | 2.9 |

On obtient 1990 g d'un produit jaune clair, mi-liquide mi-solide (entièrement liquide à 45°C), dont l'analyse CPV donne les résultats suivants:

$C_nF_{2n+1}-CH_2CH_2-NH-CH_2CH_2OH$ .................. 69,3 %

$C_nF_{2n-1}-CFH-CH_2CH_2-NH-CH_2CH_2OH$ ............. 18,6 %

$C_nF_{2n-1}-CH_2CH_2CH_2-NH-CH_2CH_2OH$ .............. 9,8 %

b/ A 5,5 g de ce mélange d'amino-alcools fluorés saturés, on ajoute 2,05 g d'acrylate d'éthyl-2-hexyle, puis on porte le mélange à 100°C pendant 8 heures.

On obtient ainsi avec un rendement de 93% un liquide limpide orangé constitué par les esters de formules:

$$C_nF_{2n+1}C_2H_4-N(C_2H_4OH)-C_2H_4COOCH_2CH(C_2H_5)-C_4H_9\quad C_{n-1}F_{2n-1}CFHCH_2CH_2-N(C_2H_4OH)-C_2H_4COOCH_2CH(C_2H_5)-C_4H_9$$

et

$$C_{n-1}F_{2n-1}CH_2CH_2CH_2-N(C_2H_4OH)-C_2H_4COOCH_2CH(C_2H_5)-C_4H_9$$

Exemple 13

On répète l'exemple 12-b, mais en remplaçant l'acrylate d'éthyl-2 hexyle par 3,3 g d'acrylate de lauryle et en utilisant 6,87 g du mélange d'amino-alcools fluorés saturés obtenu à l'exemple 12-a.

Le mélange d'esters de lauryle ainsi obtenu (rendement: 92%) se présente sous forme d'un liquide limpide jaune.

Exemple 14

Dans un erlen de 250 ml surmonté d'un réfrigerant, on ajoute 1,56 g (0,85 équivalent molaire) d'acrylate de n-butyle à 6,73 g du mélange d'amino-alcools fluorés saturés obtenu à l'exemple 12-a dans 12 g d'un mélange (3/1) d'éther et d'acétonitrile. On laisse ensuite le mélange sous agitation à température ambiante pendant 96 heures, puis on élimine les solvants par distillation sous vide.

On récupère ainsi 7,5 g d'un liquide limpide jaune constitué, dans une proportion d'environ 20% en moles, par les amino-alcools fluorés saturés de départ et, dans une proportion d'environ 80% en moles, par les esters de butyle de ces amino-alcools.

TESTS ANTI-USURE

Le pouvoir anti-usure de compositions lubrifiantes contenant comme huile de base l'huile minérale 200 Neutral Solvent et comme additif un composé à chaîne fluorée selon l'invention a été déterminé à l'aide de la machine 4 billes EP de SHELL dont la description figure dans "Annual Book of ASTM Standards", Part 24 (1979), pages 680 à 688.

Le test consiste à faire tourner une bille de 12 mm de diamètre avec une vitesse de rotation de 1 500 tr/min sur trois autres billes maintenues immobiles et couvertes de lubrifiant à étudier. Une charge de 40 ou 70 daN est appliquée par un système de levier qui pousse les trois billes fixes vers a bille supérieure placée dans un mandrin.

L'efficacité anti-usure d'un lubrifiant est déterminé par la valeur moyenne des diamètres des empreintes d'usure sur les trois billes fixes, après une heure de fonctionnement.

Le tableau I suivant rassemble les résultats obtenus avec différents additifs fluorés selon l'invention, identifiés sous la forme Fx où x correspond au numero de l'exemple décrivant la préparation de l'additif fluoré qui, dans tous les cas, a été testé à une teneur pondérale de 0,1%.

TABLEAU I

| Additif fluoré | Diamètre d'empreinte d'usure en mm pour une charge appliquée de 40 daN | 70 daN |
|---|---|---|
| Néant (témoin) | 1,44 | 2,37 |
| F 1 | 0,72 | 0,78 |
| F 2 | 0,65 | 0,79 |
| F 3 | 0,66 | 0,72 |
| F 4 | 0,62 | 0,73 |
| F 5 | 0,64 | 0,81 |
| F 6 | 0,41 | 0,72 |
| F 7 | 0,45 | 0,68 |
| F 9 | 0,51 | 0,63 |
| F 10 | 0,41 | 0,69 |
| F 14 | 0,39 | 0,55 |

TESTS DE STABILITE THERMIQUE

Afin d'apprécier leur comportement dans des conditions proches de celles existant dans un moteur, les composés fluorés selon l'invention ont été soumis à une analyse thermique gravimétrique sous air. Ce test consiste à soumettre un échantillon de produit à une élévation de température (2°C/min) sous un débit d'air de 10 l/h et à enregistrer les pourcentages de perte de poids à 200, 250 et 300°C.

Le tableau II suivant rassemble les résultats obtenus. A titre comparatif, on a indiqué au début du tableau le comportement des composés fluorés suivants préconisés dans la technique antérieure (brevet FR 2 520 377).

<u>P 1</u> : $C_8F_{17}-C_2H_4-NH-C_2H_4OH$

<u>P 2</u> : $C_nF_{2n+1}-C_2H_4-NH-C_2H_4OH$

(n défini comme à l'exemple 5 ci-dessus)

<u>T A B L E A U  II</u>

| Additif fluoré | Perte de poids (%) à : 200°C | 250°C | 300°C |
|---|---|---|---|
| P 1 | 80 | 94,6 | 97 |
| P 2 | 78,7 | 96,5 | 98,2 |
| F 3 | 16,3 | 85,5 | 96,9 |
| F 4 | 7,7 | 33,1 | 83,7 |
| F 5 | 25 | 87,5 | 95 |
| F 6 | 15 | 71,2 | 94,6 |
| F 7 | 16,2 | 70 | 91,8 |
| F 8 | 5,6 | 24,3 | 81,8 |
| F 11 | 21,2 | 71,9 | 94,4 |
| F 12 | 12,1 | 48,1 | 93,1 |
| F 13 | 5,8 | 23,1 | 75,6 |

**Revendications pour les Etats contractants: AT BE CH DE FR GB GR IT LI LU NL SE**

1. Composés polyfluorés, caractérisés en ce qu'ils répondent à la formule générale:

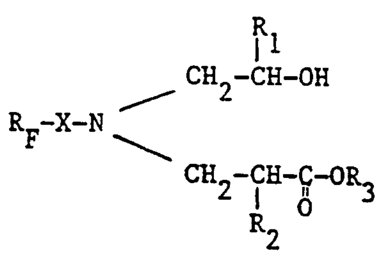

(I)

dans laquelle:

$R_F$ désigne un radical perfluoré,

X représente un enchaînement bivalent $—(CH_2CF_2)_a(CH_2)_b—$, $—CF=CHCH_2—$ ou $—CFHCH_2CH_2$, a étant

un nombre entier allant de 0 à 10, et b un nombre entier pouvant aller de 1 à 4, mais égal à 2 quand a est différent de 0,

$R_1$ représente un atome d'hydrogène ou un radical alkyle, linéaire ou ramifié, contenant de 1 à 12 atomes de carbone,

$R_2$ représente un atome d'hydrogène ou un radical méthyle, et

$R_3$ représente un radical alkyle, linéaire ou ramifié, contenant de 1 à 24 atomes de carbone.

2. Composés selon la revendication 1, dans lesquels $R_F$ est un radical perfluoroalkyle, linéaire ou ramifié, contenant de 2 à 20 atomes de carbone et $R_2$ est un atome d'hydrogène.

3. Composés selon la revendication 1, dans lesquels $R_F$ est un radical perfluoroalkyle linéaire contenant de 6 à 16 atomes de carbone, X est un enchaînement $—CH_2CH_2—$, $—CF{=}CHCH_2—$ ou $—CFHCH_2CH_2—$, $R_1$ et $R_2$ sont des atomes d'hydrogène, et $R_3$ est un radical alkyle contenant de 8 à 18 atomes de carbone.

4. Mélanges de composés selon l'une des revendications 1 à 3, dans lesquels les groupements $R_F$, X et/ou $R_3$ sont différents.

5. Mélanges, caractérisés en ce qu'ils contiennent un ou plusieurs composés selon l'une des revendications 1 à 3 et jusqu'à 50% d'un ou plusieurs amino-alcools de formule:

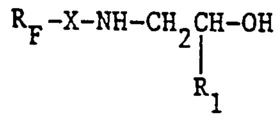

(II)

dans laquelle les symboles $R_F$, X et $R_1$ ont les mêmes significations que dans la revendication 1.

6. Procédé de préparation de composés polyfluorés ou de mélanges de tels composés, caractérisé en ce que l'on condense un amino-alcool de formule:

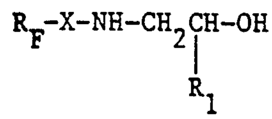

(II)

ou un mélange de tels amino-alcools, sur un ester acrylique de formule:

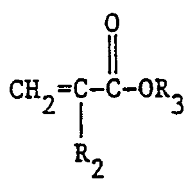

(III)

ou un mélange de tels esters, les symboles $R_F$, X, $R_1$, $R_2$ et $R_3$ ayant les mêmes significations que dans la revendication 1.

7. Procédé selon la revendication 6, dans lequel la condensation est effectuée à une température comprise entre 20 et 100°C, de préférence entre 20 et 80°C, au sein d'un alcool inférieur ($C_1$—$C_4$).

8. Procédé de préparation de composés selon la revendication 1 dans lesquels $R_3$ est un radical alkyle à longue chaîne ($C_5$—$C_{24}$), caractérisé en ce que l'on transestérifie à l'aide d'un alcool à longue chaîne un composé selon la revendication 1 dans lequel $R_3$ est un radical alkyle inférieur ($C_1$—$C_4$).

9. Utilisation des composés polyfluorés et des mélanges selon l'une des revendications 1 à 5 comme additifs anti-usure pour lubrifiants.

10. Lubrifiants, caractérisés en ce qu'ils contiennent un composé polyfluoré ou un mélange de composés polyfluorés selon l'une des revendications 1 à 5.

11. Lubrifiants selon la revendication 10, dans lesquels la teneur en composé(s) polyfluoré(s) est d'au moins 0,01% en poids et, de préférence, comprise entre 0,05 et 0,5%.

12. Lubrifiants selon la revendication 10 ou 11, dans lesquels le ou les composés polyfluorés sont associés aux additifs classiques.

**Revendications pour l'Etat contractant: ES**

1. Procéde de préparation des composés polyfluorés répondant à la formule générale:

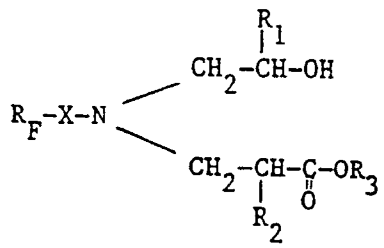

(I)

dans laquelle:

$R_F$ désigne un radical perfluoré,

X représente un enchaînement bivalent $-(CH_2CF_2)_a(CH_2)_b-$, $-CF=CHCH_2-$ ou $-CFHCH_2CH_2$, a étant un nombre entier allant de 0 à 10, et b un nombre entier pouvant aller de 1 à 4, mais égal à 2 quand a est différent de 0,

$R_1$ représente un atome d'hydrogène ou un radical alkyle, linéaire ou ramifié, contenant de 1 à 12 atomes de carbone,

$R_2$ représente un atome d'hydrogène ou un radical méthyle, et

$R_3$ représente un radical alkyle, linéaire ou ramifié, contenant de 1 à 24 atomes de carbone,

ou de mélanges contenant un ou plusieurs de ces composés et jusqu'à 50% d'un ou plusieurs amino-alcools de formule:

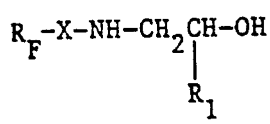

(II)

dans laquelle les symboles $R_F$, X et $R_1$ ont les mêmes significations que ci-dessus, caractérisé en ce que l'on condense un amino-alcool de formule:

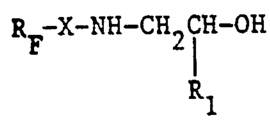

(II)

ou un mélange de tels amino-alcools, sur un ester acrylique de formule:

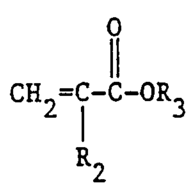

(III)

ou un mélange de tels esters, les symboles $R_F$, X, $R_1$, $R_2$ et $R_3$ ayant les mêmes significations que ci-dessus.

2. Procédé selon la revendication 1, dans lesquel $R_F$ est un radical perfluoroalkyle, linéaire ou ramifié, contenant de 2 à 20 atomes de carbone et $R_2$ est un atome d'hydrogène.

3. Procédé selon la revendication 1, dans lesquel $R_F$ est un radical perfluoroalkyle linéaire contenant de 6 à 16 atomes de carbone, X est un enchaînement $-CH_2CH_2-$, $-CF=CHCH_2-$ ou $-CFHCH_2CH_2-$, $R_1$ et $R_2$ sont des atomes d'hydrogène, et $R_3$ est un radical alkyle contenant de 8 à 18 atomes de carbone.

4. Procédé selon l'une des revendications 1 à 3, dans lequel la condensation est effectuée à une température comprise entre 20 et 100°C, de préférence entre 20 et 80°C, au sein d'un alcool inférieur ($C_1$—$C_4$).

5. Procédé de préparation de composés selon la revendication 1 dans lesquels $R_3$ est un radical alkyle à longue chaîne ($C_5$—$C_{24}$), caractérisé en ce que l'on transestérifie à l'aide d'un alcool à longue chaîne un composé selon la revendication 1 dans lequel $R_3$ est un radical alkyle inférieur ($C_1$—$C_4$).

6. Utilisation des composés polyfluorés (I) et de leurs mélanges tels que définis dans la revendication 1, comme additifs anti-usure pour lubrifiants.

7. Utilisation selon la revendication 6, caractérisé en ce que la teneur du lubrifiant en composé(s) polyfluoré(s) est d'au moins 0,01% en poids et, de préférence, comprise entre 0,05 et 0,5%.

8. Utilisation selon la revendication 6 ou 7, caractérisée en ce que le ou les composés polyfluorés sont associés aux additifs classiques.

**Patentansprüche für die Vertragsstaaten: AT BE CH DE FR GB GR IT LI LU NL SE**

1. Polyfluorierte Verbindungen, dadurch gekennzeichnet, daß sie der allgemeinen Formel

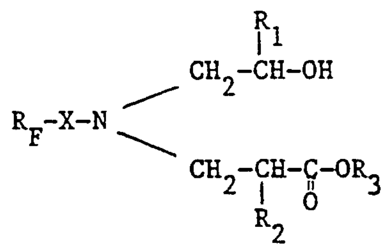

(I)

worin

$R_F$ einen perfluorierten Rest bezeichnet,

X eine zweiwertige Verknüpfung $-(CH_2CF_2)_a(CH_2)_b-$, $-CF=CHCH_2-$ oder $-CFHCH_2CH_2-$ bedeutet, a eine ganze Zahl von 0 bis 10 ist und b eine ganze Zahl von 1 bis 4 sein kann, aber gleich 2, wenn a von 0 verschieden ist,

$R_1$ ein Wasserstoffatom oder einen geradkettigen oder verzweigten, 1 bis 12 Kohlenstoffatome enthaltenden Alkylrest,

$R_2$ ein Wasserstoffatom oder einen Methylrest und

$R_3$ einen geradkettigen oder verzweigten, 1 bis 24 Kohlenstoffatome enthaltenden Alkylrest bedeutet.

2. Verbindungen nach Anspruch 1, worin $R_F$ ein geradkettiger oder verzweigter, 2 bis 20 Kohlenstoffatome enthaltender Perfluoralkylrest und $R_2$ ein Wasserstoffatom ist.

3. Verbindungen nach Anspruch 1, worin $R_F$ ein geradkettiger, 6 bis 16 Kohlenstoffatome enthaltender Perfluoralkylrest, X eine Verknüpfung $-CH_2CH_2-$, $-CF=CHCH_2-$ oder $-CFHCH_2CH_2-$ ist, $R_1$ und $R_2$ Wasserstoffatome sind und $R_3$ ein 8 bis 18 Kohlenstoffatome enthaltender Alkylrest ist.

4. Gemische der Verbindungen nach einem der Ansprüche 1 bis 3, in denen die $R_F$ X und/oder $R_3$ Gruppen verschieden sind.

5. Gemische, dadurch gekennzeichnet, daß sie eine oder mehrere Verbindungen nach einem der Ansprüche 1 bis 3 und bis zu 50% eines oder mehrerer Aminoalkohole der Formel

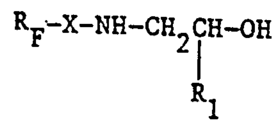

(II)

enthalten, worin $R_F$, X und $R_1$ die selben Bedeutungen wie in Anspruch 1 haben.

6. Verfahren zur Herstellung von fluorhaltigen Produkten oder von Gemischen solcher Produkte, dadurch gekennzeichnet, daß man einen Aminoalkohol der Formel

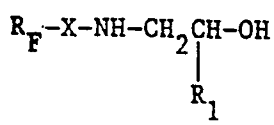

(II)

oder ein Gemisch solcher Aminoalkohole mit einem Acrylsäureester der Formel

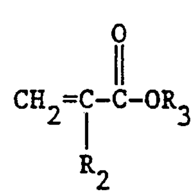

(III)

oder einem Gemisch solcher Ester kondensiert, worin $R_F$, X, $R_1$ und $R_3$ die selben Bedeutungen wie in Anspruch 1 haben.

7. Verfahren nach Anspruch 6, worin die Kondensation bei einer Temperatur zwischen 20 und 100°C

und vorzugsweise zwischen 20 und 80°C in einem niederen ($C_1$—$C_4$) alkohol durchgeführt wird.

8. Verfahren zur Herstellung von Verbindungen nach Anspruch 1, worin $R_3$ ein langkettiger ($C_5$—$C_{24}$) Alkylrest ist, dadurch gekennzeichnet, daß man mit einem langkettigen Alkohol eine Verbindung nach Anspruch 1, worin $R_3$ ein niederer ($C_1$—$C_4$) Alkylrest ist, umestert.

9. Verwendung der polyfluorierten Verbindungen und der Gemische nach einem der Ansprüche 1 bis 5 als Antiverschleißadditive für Schmiermittel.

10. Schmiermittel, dadurch gekennzeichnet, daß sie eine polyfluorierte Verbindung oder ein Gemisch aus polyfluorierten Verbindungen nach einem der Ansprüche 1 bis 5 enthalten.

11. Schmiermittel nach Anspruch 10, dadurch gekennzeichnet, daß der Gehalt an polyfluorierter(en) Verbindung(en) mindestens 0,01 Gew.% und vorzugsweise zwischen 0,05 und 0,5% beträgt.

12. Schmiermittel nach Anspruch 10 oder 11, dadurch gekennzeichnet, daß in ihnen die polyfluorierte(n) Verbindung(en) mit klassischen Additiven verbunden ist(sind).

**Patentansprüche für den Vertragsstaat: ES**

1. Verfahren zur Herstellung von polyfluorierten Verbindungen der allgemeinen Formel

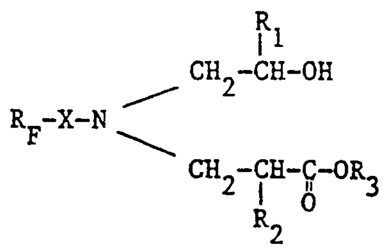

(I)

worin

$R_F$ einen perfluorierten Rest bezeichnet,

X eine zweiwertige Verknüpfung —$(CH_2CF_2)_a(CH_2)_b$—, —CF=$CHCH_2$— oder —$CFHCH_2CH_2$— bedeutet, a eine ganze Zahl von 0 bis 0 ist und b eine ganze Zahl von 1 bis 4 sein kann, aber gleich 2, wenn a von 0 verschieden ist,

$R_1$ ein Wasserstoffatom oder einen geradkettigen oder verzweigten, 1 bis 12 Kohlenstoffatome enthaltenden Alkylrest,

$R_2$ ein Wasserstoffatom oder einen Methylrest und

$R_3$ einen geradkettigen oder verzweigten, 1 bis 24 Kohlenstoffatome enthaltenden Alkylrest bedeutet, oder von Gemischen, die eine oder mehrere dieser Verbindungen und bis zu 50% eines oder mehrerer Aminoalkohole der Formel

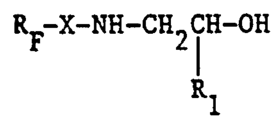

(II)

enthalten, worin $R_F$, X und $R_1$ die selben Bedeutungen wie zuvor haben, dadurch gekennzeichnet, daß man einen Aminoalkohol der Formel

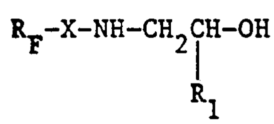

(II)

oder ein Gemisch solcher Aminoalkohole mit einem Acrylsäureester der Formel

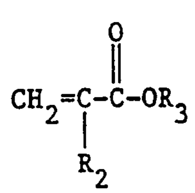

(III)

oder einem Gemisch solcher Ester kondensiert, worin $R_F$, X, $R_1$ und $R_3$ die selben Bedeutungen wie zuvor haben.

2. Verfahren nach Anspruch 1, worin $R_F$ ein geradkettiger oder verzweigter, 2 bis 20 Kohlenstoffatome enthaltender Perfluoralkylrest und $R_2$ ein Wasserstoffatom ist.

3. Verfahren nach Anspruch 1, worin $R_F$ ein geradkettiger, 6 bis 16 Kohlenstoffatome enthaltender Perfluoralkylrest, X eine Verknüpfung $—CH_2CH_2—$, $—CF{=}CHCH_2—$ oder $—CFHCH_2CH_2—$ ist, $R_1$ und $R_2$ Wasserstoffatome sind und $R_3$ ein 8 bis 18 Kohlenstoffatome enthaltender Alkylrest ist.

4. Verfahren nach einem der Ansprüche 1 bis 3, worin die Kondensation bi einer Temperatur zwischen 20 und 100°C und vorzugsweise zwischen 20 und 80°C in einem niederen ($C_1$—$C_4$) Alkohol durchgeführt wird.

5. Verfahren zur Herstellung von Verbindungen nach Anspruch 1, worin $R_3$ ein langkettiger ($C_5$—$C_{24}$) Alkylrest ist, dadurch gekennzeichnet, daß man mit einem langkettigen Alkohol eine Verbindung nach Anspruch 1, worin $R_3$ ein niederer ($C_1$—$C_4$) Alkylrest ist, umestert.

6. Verwendung der polyfluorierten Verbindungen (I) und ihrer wie in Anspruch 1 definierten Gemische als Antiverschleißadditive für Schmiermittel.

7. Verwendung nach Anspruch 6, dadurch gekennzeichnet, daß der Gehalt des Schmiermittels an polyfluorierter(n) Verbindung(en) wenigstens 0,01 Gewichtsprozent und vorzugsweise zwischen 0,05 und 0,5% beträgt.

8. Verwendung nach Anspruch 6 oder 7, dadurch gekennzeichnet, daß die polyfluorierte(n) Verbindung(en) an klassische Additive gebunden ist (sind).

**Claims for the Contracting States: AT BE CH DE FR GB GR IT LI LU NL SE**

1. Polyfluoro compounds, characterized in that they correspond to the general formula:

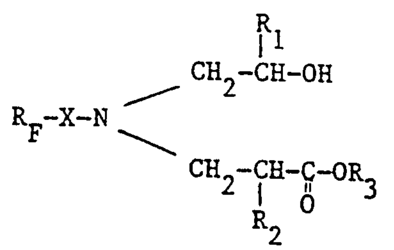

(I)

in which:

$R_F$ denotes a perfluorinated radical,

X denotes a divalent $—(CH_2CF_2)_a(CH_2)_b—$, $—CF{=}CHCH_2—$ or $—CFHCH_2CH_2—$ chain sequence, a being an integer ranging from 0 to 10 and b an integer capable of ranging from 1 to 4 but equal to 2 when a is other than 0,

$R_1$ denotes a hydrogen atom or a linear or branched alkyl radical containing from 1 to 12 carbon atoms,

$R_2$ denotes a hydrogen atom or a methyl radical, and

$R_3$ denotes a linear or branched alkyl radical containing from 1 to 24 carbon atoms.

2. Compounds according to Claim 1, in which $R_F$ is a linear or branched perfluoroalkyl radical containing from 2 to 20 carbon atoms and $R_2$ is a hydrogen atom.

3. Compounds according to Claim 1, in which $R_F$ is a linear perfluoroalkyl radical containing from 6 to 16 carbon atoms, X is a $—CH_2CH_2—$, $—CF{=}CHCH_2—$ or $—CFHCH_2CH_2—$ chain sequence, $R_1$ and $R_2$ are hydrogen atoms and $R_3$ is an alkyl radical containing from 8 to 18 carbon atoms.

4. Mixtures of compounds according to one of Claims 1 to 3, in which the groups $R_F$, X and/or $R_3$ are different.

5. Mixtures, characterized in that they contain one or more compounds according to one of Claims 1 to 3 and up to 50% of one or more aminoalcohols of formula:

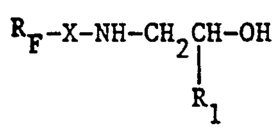

(II)

in which the symbols $R_F$, X and $R_1$ have the same meanings as in Claim 1.

6. Process for the preparation of polyfluoro compounds or of mixtures of such compounds, characterized in that an aminoalcohol of formula:

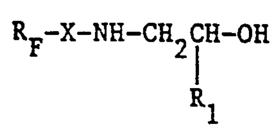

(II)

or a mixture of such aminoalcohols, is condensed with an acrylic ester of formula:

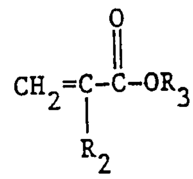 (III)

or a mixture of such esters, the symbols $R_F$, X, $R_1$, $R_2$ and $R_3$ having the same meanings as in claim 1.

7. Process according to Claim 6, in which the condensation is carried out a temperature of between 20 and 100°C, preferably between 20 and 80°C, in a lower ($C_1$—$C_4$) alcohol.

8. Process for the preparation of compounds according to Claim 1, in which $R_3$ is a long-chain ($C_5$—$C_{24}$) alkyl radical, characterized in that a compound according to Claim 1 in which $R_3$ is a lower ($C_1$—$C_4$) alkyl radical is transesterified with the aid of a long-chain alcohol.

9. Use of the polyfluoro compounds and of the mixtures according to one of Claims 1 to 5 as antiwear additives for lubricants.

10. Lubricants characterized in that they contain a polyfluoro compound or a mixture of polyfluoro compounds according to one of Claims 1 to 5.

11. Lubricants according to Claim 10, in which the content of polyfluoro compound(s) is at least 0.01% by weight and preferably between 0.05 and 0.5%.

12. Lubricants according to Claim 10 or 11, in which the polyfluoro compound(s) is(are) coupled with traditional additives.

**Claims for the Contracting State: ES**

1. Process for the preparation of polyfluoro compounds corresponding to the general formula:

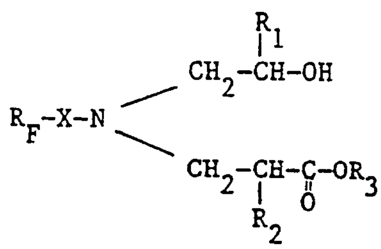 (I)

in which:

$R_F$ denotes a perfluorinated radical,

X denotes a divalent —$(CH_2CF_2)_a(CH_2)_b$—, —$CF{=}CHCH_2$— or —$CFHCH_2CH_2$— chain sequence, a being an integer ranging from 0 to 10 and b an integer capable of ranging from 1 to 4 but equal to 2 when a is other than 0,

$R_1$ denotes a hydrogen atom or a linear or branched alkyl radical containing from 1 to 12 carbon atoms,

$R_2$ denotes a hydrogen atom or a methyl radical, and

$R_3$ denotes a linear or branched alkyl radical containing from 1 to 24 carbon atoms.

or of mixtures containinig one or more of these compounds and up to 50% of one or more aminoalcohols of formula:

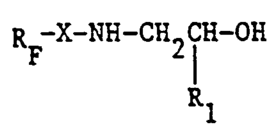 (II)

in which the symbols $R_F$, X and $R_1$ have the same meanings as above, characterized in that an aminoalcohol of formula:

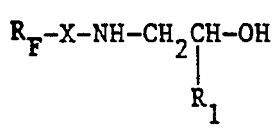 (II)

or a mixture of such aminoalcohols, is condensed with an acrylic ester of formula:

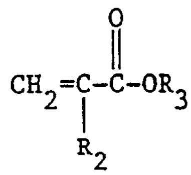

(III)

or a mixture of such esters, the symbols $R_F$, X, $R_1$, $R_2$ and $R_3$ having the same meanings as above.

2. Process according to Claim 1, in which $R_F$ is linear or branched perfluoroalkyl radical containing from 2 to 20 carbon atoms and $R_2$ is a hydrogen atom.

3. Process according to Claim 1, in whch $R_F$ is a linear perfluoroalkyl radical containing from 6 to 16 carbon atoms, X is a $—CH_2CH_2—$, $—CF{=}CHCH_2—$ or $—CFHCH_2CH_2—$ chain sequence, $R_1$ and $R_2$ are hydrogen atoms and $R_3$ is an alkyl radical containing from 8 to 18 carbon atoms.

4. Process according to one of Claims 1 to 3, in which the condensation is carried out at a temperature of between 20 and 100°C, preferably between 20 and 80°C, in a lower ($C_1—C_4$) alcohol.

5. Process for the preparation of compounds according to Claim 1, in which $R_3$ is a long-chain ($C_5—C_{24}$) alkyl radical, characterized in that a compound according to Claim 1 in which $R_3$ is a lower ($C_1—C_4$) alkyl radical is transesterified with the aid of a long-chain alcohol.

6. Use of the polyfluoro compounds (I) and of their mixtures such as defined in Claim 1, as antiwear additives for lubricants.

7. Use according to Claim 6, characterized in that the polyfluoro compound(s) content of the lubricant is at least 0.01% by weight and preferably between 0.05 and 0.5%.

8. Use according to Claim 6 or 7, characterized in that the polyfluoro compound(s) is(are) coupled with traditional additives.